# FASCICULE DE BREVET EUROPEEN

(11) **EP 0 520 890 B1**
(45) Date de publication et mention de la délivrance du brevet: **11.12.1996**
(21) Numéro de dépôt: 92401790.8
(22) Date de dépôt: 25.06.1992
(51) Int. Cl.: A23K 1/16, A23K 1/18, A61K 9/50

(54) **Composition sous forme de pellets contenant des principes actifs non protégés vis-à-vis de la panse des ruminants associés à des principes actifs protégés contre la dégradation dans la panse des ruminants**
Pelletförmige Zusammensetzung, die Wirkstoffe enthält, die nicht geschützt sind gegen Abbau im Pansen von Wiederkäuern zusammen mit Wirkstoffen, die geschützt sind gegen Abbau im Pansen von Wiederkäuern
Pellet composition comprising active substances unprotected in the rumen of ruminants, in combination with active substances protected against degradation in the rumen of ruminants

(30) Priorité: 28.06.1991 FR 9108044
(43) Date de publication de la demande: 30.12.1992
(73) Titulaire: RHONE-POULENC NUTRITION ANIMALE, 03600 Commentry (FR)
(72) Inventeur: Laffay, Jean-Claude, F-03420 Saint Priest (FR); Ruel, Jacques, F-95210 Saint Gratien (FR); Sabatier, Alain, F-75013 Paris (FR)
(74) Mandataire: Le Pennec, Magali

(56) Documents cités:
- EP-A- 0 100 974
- EP-A- 0 180 743
- EP-A- 0 202 409
- EP-A- 0 246 863
- EP-A- 0 387 597
- EP-A- 0 387 597
- EP-A- 0 399 819
- EP-A- 0 399 819
- EP-A- 0 426 463
- EP-A- 0 437 388
- EP-A- 0 437 388
- EP-A- 0 437 388
- AU-A- 548 773
- US-A- 4 048 268

## Description

La présente invention concerne un procédé d'incorporation dans des pellets de principes actifs. Elle concerne plus particulièrement l'incorporation dans des pellets à la fois de principes actifs non protégés vis à vis du rumen associés à des principes actifs protégés vis à vis du milieu existant dans la panse des ruminants. Elle concerne encore plus particulièrement la préparation de pellets destinés à être incorporés à l'alimentation des ruminants.

On entend par "pellet" un granulé nutritif pour les animaux obtenu soit par extrusion à travers une filière d'un mélange d'aliments soit par une autre technique.

Ils se présentent sous forme de bâtonnets de préférence cylindrique présentant notamment des dimensions moyennes d'une longueur de 4 à 100 mm et d'un diamètre de 2 à 30 mm. Ils présentent de préférence des dimensions moyennes de 4 à 30 mm de long et de 2 à 10 mm de diamètre. Ils sont largement utilisés par les agriculteurs, car ils sont faciles à manipuler, à administrer et non poussiérants.

Le problème que cherche à résoudre la présente invention consiste à incorporer dans des "pellets" destinés à l'alimentation animale des composés contenant des principes actifs utiles à leur alimentation et/ou à la thérapeutique de ces animaux, mais qui ne peuvent subir la pelletisation du fait de leur sensibilité à la température, à la pression et au cisaillement. En effet, les appareils de pelletisation sont des appareils qui pour permettre la mise en forme des aliments utilisent à la fois la chaleur et la pression en réalisant le forçage d'un mélange alimentaire à travers une filière en présence de vapeur d'eau.

Ce mélange est passé à travers une plaque perforée (la filière) par le moyen d'une presse qui force la masse à extruder à travers les orifices de la plaque. A la sortie de la filière, les cylindres obtenus sont coupés par un moyen mécanique ou spontanément. Lors du passage forcé à travers la filière, les produits à faible résistance thermique ou mécanique subissent une dégradation due à la pression exercée et à la chaleur provoquée à la fois par le frottement et par l'addition de vapeur d'eau favorable à la cohésion des divers ingrédients farineux des aliments de base.

Ce problème a déjà été évoqué dans diverses antériorités. On peut citer parmi ces antériorités la demande de brevet européen publiée sous la numéro EP 231 817 qui décrit un procédé de préparation "d'aliments agglomérés" à base de vitamines ou de composés indispensables à la santé humaine ou animale. Ce problème de dégradation est particulièrement bien expliqué dans le commentaire de l'art antérieur de cette demande de brevet EP 231.817 où les composés à faible résistance thermique ou mécanique sont toujours au moins partiellemnt détruits, lors de leur mise en forme, quand ils sont mélangés à de la mélasse et subissent, ensuite, l'action de la vapeur afin d'assurer la cohésion du produit final

La demande de brevet EP 231 817 a permis de résoudre le problème de fabrication de ces "aliments agglomérés" par pulvérisation sur un noyau à base d'aliments agglomérés d'une solution ou d'une suspension de la vitamine ou des vitamines ou du médicament et éventuellement d'un corps gras. Ce procédé ne permet qu'un enrobage du corps de base par la vitamine avec ou sans corps gras. Ledit procédé est facile à mettre en oeuvre à partir de produits pulvérulents ou liquides tels que les vitamines ou les médicaments car ces produits sont faciles à mettre en oeuvre sous forme de solution ou d'une suspension. Il n'en est pas de même lorsque l'on forme des "granulés nutritifs agglomérés" dans lesquels la matière sensible à la chaleur et/ou à la compression se présente elle-même sous forme de granulés de diamètre moyen d'environ 0,3 à 5 mm de préférence de 0,5 à 3 mm ce qui est le cas dans la présente invention.

Il est également décrit dans le brevet français publié sous le numéro FR 2 338 653 un procédé de préparation de produits alimentaires sur lesquels sont pulvérisés une suspension d'enzyme de préférence de protéases dans une matière grasse liquide ou fondue. Ce procédé apporte le même enseignement que la demande européenne préalablement évoquée et ne permet d'obtenir que des "pellets" sur lesquels la substance thermosensible est déposée en périphérie par un phénomène de pulvérisation.

Il est encore décrit dans les brevets EP-A-0100974, EP-A-0399819, AU-B-0548773 des complexes de principe actif et d'un élement divers qui sont stables à la chaleur et à la pression et pour certains d'entre eux qui sont stables vis-à-vis du rumen. Sont ainsi décrits respectivement des complexes avec le zinc, avec des farines, avec de la montmorillonite, ces complexes sont tous stables à la chaleur donc pelletisables, seul le complexe avec des farines spéciales n'apporte pas une stabilité vis-à-vis du rumen.

Il est également décrit dans le brevet EP-A-0387597 l'inclusion de principe actif dans une matrice de lipides gélifiés qui permet auxdits principes actifs de présenter une stabilité vis-à-vis du rumen et aux dits granulés de présenter une résistance améliorée lors de la pelletization.

Il est également décrit dans la demande de brevet déposée sous le numéro FR 89.17305 de nouvelles compositions sous forme de "pellets" contenant des principes actifs granulaires protégés vis à vis de la dégradation dans le rumen libérant lesdits principes actifs dans la caillette et/ou l'intestin, compositions qui sont obtenues par mélange du principe actif protégé, sous forme granulaire, et d'un agent liant solubilisable, réticulable ou fusible et éventuellemnt d'un agent délitant et/ou d'une charge. Cette demande de brevet n'inclut pas d'autres agents thérapeutiques ou nutritionnels que ceux qui se présentent sous forme granulaire et qui sont protégés vis à vis de la dégradation du rumen.

Le problème que cherche à résoudre la présente invention est la mise sous forme de "pellets", directement assimilables par les animaux, d'additifs médicamenteux ou nutritionnels protégés vis à vis de la dégradation dans la panse des ruminants tels que les aminoacides, les vitamines, associés à des composés nutritionnels ou médicamenteux présents sous une forme non protégée.

En ce qui concerne le premier type d'additifs c'est à dire les additifs protégés vis à vis de la dégradation dans le rumen, on peut d'abord citer les aminoacides tels que la méthionine ou la lysine. Il est connu par exemple, que certains aminoacides sont essentiels dans l'alimentation des ruminants car ils sont limitants dans l'apport nutritionnel journalier. Il en est ainsi notamment de la méthionine et de la lysine. Ces substances, lorsqu'elles sont administrées aux ruminants par voie orale sont détruites dans la panse par l'action des enzymes digestives et des microorganismes présents dans cet organe. Il a été découvert que pour que ces composés soient utilisables et bénéfiques pour l'animal, il fallait les protéger par une substance leur permettant de traverser le rumen sans dommage mais délitable au niveau ou après la caillette de façon à libérer la substance active dans l'intestin et permettre ainsi son passage dans l'organisme.

Généralement, les compositions d'enrobage connues sont constituées de l'association d'une substance sensible aux variations du PH, choisie en particulier parmi des copolymères basiques synthétiques, avec des substances hydrophobes qui peuvent être choisies, par exemple, parmi les acides gras ou leurs dérivés et les polymères hydrophobes. De telles compositions sont décrites, par exemple, dans les brevets français FR 78 23966 (2 401 620), FR 78 23968 (2 401 621) ou FR 81 18954 (2 514 261).

Parmi les principaux groupes d'agents d'enrobage, on peut citer à titre d'exemples les copolymères de la vinyl pyridine et du styrène avec une substance hydrophobe de préférence l'acide stéarique et/ou un polymère non hydrosoluble, par exemple l'éthylcellulose.

On peut aussi citer le groupe d'agents d'enrobage à digestion enzymatique, tels que le chitosan et/ou la zéïne, associé à une substance hydrophobe de préférence l'acide stéarique et éventuellement à un polymère non hydrosoluble de préférence l'éthylcellulose.

Les granulés de principes actifs protégés obtenus se présentent sous forme de particules plus ou moins sphériques ayant un diamètre moyen compris entre 0,3 et 5 mm, de préférence d'environ 2 mm.

Il est inenvisageable de pouvoir pulvériser des granulés de cette dimension sur des "pellets" de matière nutritive. Ces granulés ne peuvent pas non plus être introduits dans des appareils de pelletisation car l'enrobant subit des aggressions telles que l'abrasion, le cisaillement, l'élévation de température, l'adjonction de vapeur d'eau et la pression qui entraînent une dégradation au moins partielle de l'enrobant et donc une non-stabilité des principes actifs dans le rumen lors de l'ingestion par l'animal.

Il semblait donc impossible de faire ingérer aux ruminants lesdits principes actifs protégés autrement qu'en les dispersant dans l'aliment ce qui pose des problèmes d'homogénéité, de concentration et de distribution.

L'invention décrite dans la demande de brevet EP 437 388 a permis d'atteindre cet objectif. Elle consiste à préparer des "pellets spéciaux' contenant des principes actifs protégés contre la dégradation dans le rumen, utilisés pour la supplémentation alimentaire et/ou médicamenteuse des ruminants caractérisés en ce qu'ils sont obtenus par mélange du principe actif protégé sous forme de granulé d'un liant choisi parmi les liants solubilisables, reticulables, les liant fusibles et éventuellement d'un agent délitant et/ou d'une charge.

Les principes actifs protégés contre l'action du rumen utilisables sous cette forme sont choisis parmi :
- les aminoacides essentiels ,leurs sels, leurs dérivés et leurs analogues tels que: la méthionine, la lysine;
- les vitamines;
- les principes médicamenteux tels que les antibiotiques.

Les agents liants utilisables dans le cadre de la présente invention sont choisis de préférence parmi les matières alimentaires permettant une transformation liquide/solide de telle sorte qu'une forme définie proche de celle du "pellet" alimentaire puisse lui être conférée et figée.

Deux classes d'agents liants alimentaires peuvent être utilisés :
- les agents liants utilisés en milieu solvant ou dispersant,
- les agents fusibles.

Parmi les agents liants utilisés en milieu solvant ou dispersant, on peut citer :
- la classe des hydrocolloïdes tels que notamment les dérivés hydrosolubles de la cellulose tels que : la carboxyméthyl cellulose, l'hydroxypropyl cellulose, l'hydroxyéthyl cellulose, l'hydroxyméthyl cellulose;
- la classe des polysaccharides naturels ou synthétiques tels que: la gomme arabique, la gomme adragante, les carraghénates, les dextrines, l'amidon, la gomme xanthane, les alginates;
- les sucres,
- les mélasses et les vinasses,
- les lignosulfonates,
- les farines végétales ou d'algues,
- la classe des composés minéraux cristallisables tels que la chaux, le plâtre, le silicate de sodium, le carbonate de calcium et la silice,
- les gélatines,
- les protéines tannées,
- les sels de cations polyvalents des polyacides naturels ou synthétiques,
- les huiles siccatives et les mastics obtenus par association d'une huile siccative et d'une charge.

Certains liants sont utilisés avec des agents de réticulation choisis par exemple parmi: les aldéhydes pour les protéines, les sels ou oxydes de métaux di ou trivalents pour les alginates, la gomme xanthane, les mélasses,les vinasses et les autres agents de durcissement adaptés aux liants utilisés et bien connus de l'homme de l'art.

Parmi les liants fusibles, on peut citer à titre d'exemples :
- les acides et alcools gras,
- les graisses végétales et animales hydrogénées,
- les esters du glycérol,
- les paraffines,
- les cires naturelles ou synthétiques,
- les polymères synthétiques tels que les polyéthylèneglycols, l'acétate de polyvinyle...

On préfère, parmi l'ensemble des liants, utiliser les mélasses, les vinasses, les acides gras, les graisses végétales ou animales hydrogénées, le plâtre, les cires paraffiniques et les farines végétales.

Dans la classe des agents thérapeutiques ou nutritionnnels non protégés et utilisables dans le cadre de la présente invention qui donc sont intégrés dans le mélange devant subir la mise sous forme de pellets on peut citer :
- des additifs minéraux tels que le phosphore, le soufre, le magnésium, le zinc, le cuivre, le cobalt, la sodium, le potassium, le chlore, le fer, le calcium, l'iode, le molybdène, le sélénium, le nickel, le vanadium,
- des vitamines telles que les vitamines A, B, D et E,
- des levures,
- des facteurs de croissance,
- des enzymes telles que les ligninases, les cellulases telles par exemple que les glucanases, les xylanases, les α amylases, les β glucanases, les phytases,
- des éléments de la flore microbienne tels que les lactobacilles, les pediocoques, les streptocoques.
- des bactéries
- des fungi tels que par exemple les Aspergillus, les Penicillium
- des peptides tels que notamment l'hormone de croissance
- des adjuvants alimentaires tels que le bicarbonate de sodium, le sorbitol, le propylème glycol, la bétaïne, le propionate de soude, à l'exclusion d'un ou plusieurs des composés suivants : les dérivés hydrosolubles de la cellulose, les polysaccharides, les sucres, les mélasses, les vinasses, les lignosulfonates, les farines de grains ou d'algues, les composés minéraux cristallisables, les gélatines, les protéines tannées, les sels de cations polyvalent des polyacides, les huiles siccatives, les mastics, les acides ou alcools gras, les graisses animales et végétales hydrogénées, les esters de glycérol, les paraffines, les cires naturelles ou synthétiques, les polymères synthétiques, la silice, les silicates, le talc, les argiles, les carbonates de calcium, les phosphates, les résidus des industries des céréales et du bois, les tourteaux broyés, les résidus de brasserie et de fermentation, les fibres végétales cellulosiques.

On peut encore introduire dans le pellet spécial selon l'invention, des additifs qui permettent d'apporter au pellet les performances de densité, de résistance mécanique et de délitement rapide dans le rumen.

A titre d'additifs utiles pour la préparation des pellets spéciaux selon l'invention, on peut citer :
- les additifs minéraux tels que : la silice, les silicates, le talc, les argiles, les carbonates de calcium, les phosphates, les bentonites,
- les additifs issus de produits naturels tels que : les farines de grains, les résidus des industries des céréales et du bois, les tourteaux broyés, les résidus de brasserie et de fermentation diverses, des fibres végétales cellulosiques, des polysaccharides, des sucres.

Le liant complété éventuellement des additifs représente de préférence 40 à 95 % de la masse du "pellet spécial selon l'invention". La teneur en additifs minéraux ou naturels représente de 0 à 80 % en poids par rapport au liant.

Le mélange du liant et du produit protégé peut se faire en même temps que la mise en forme ou précedemment. La mise en forme peut se faire par l'intermédiaire d'une filière ou d'un moule.

La nature du liant, la température de coulée, la quantité d'additifs seront adaptées par l'homme de l'art en fonction de la qualité du pellet spécial selon l'invention désiré :
- son appétence,
- sa densité,
- sa forme,
- sa dimension,
- sa résistance mécanique,
- sa solubilité,
- son aptitude à se déliter dans le rumen.

Ils se présentent sous forme de bâtonnets de préférence cylindrique présentant notamment des dimensions moyennes de 4 à 100 mm de long et de 2 à 30 mm de diamètre.

Les pellets spéciaux selon l'invention présentent au moins trois caractéristiques qui les rendent utiles pour l'alimentation des ruminants:
1/ Ils sont miscibles en toutes proportions avec les aliments granulés généralement distribués à l'animal en supplémentation de la ration fourragère de base; c'est à dire qu'ils peuvent supporter sans démélange les différentes étapes de manutention auxquelles sont soumis les aliments granulés, et s'intègrent dans la chaine de distribution de l'aliment.
2/ La perte de protection des principes actifs protégés y est limitée lors de la mise en forme et la destruction mécanique des principes actifs protégés est réduite.
3/ Les "pellets spéciaux selon l'invention" ainsi réalisés se délitent rapidement dans le rumen, et évitent une perte de protection des principes actifs inclus, par mastication lors de la rumination. Les principes actifs protégés peuvent alors continuer librement le transit, les principes actifs non protégés sont libérés au niveau du rumen où ils peuvent agir.

L'aptitude à la miscibilité avec les aliments granulés traditionnels dépend essentiellement de la forme et de la densité des produits réalisés et ne présente pas de difficulté pour l'homme de l'art.

Le délitement des "pellets spéciaux selon l'invention" dans le rumen qui permet la restitution des principes actifs non protégés vis à vis du rumen et des granules protégés, est évalué par incubation dans le rumen de bovins fistulés au moyen de sachets en tissu de nylon de vide de maille de 350 micromètre. Le temps de délitement doit être le plus faible possible, et en particulier inférieur à 48 h.

La présente invention sera plus complètement décrite à l'aide des exemples suivants qui ne peuvent être considérés comme limitatifs, tant en ce qui concerne les principes actifs protégés, que les liants utilisés.

Les principes actifs protégés utilisés pour les exemples sont des granulés d'environ 2 mm de diamètre composés de:

### Principe actif protégé

Particules de lysine monochlorhydrate et de méthionine dans la proportion pondérale 75/25, contenant environ 12% d'un liant constitué par un mélange d'acide stéarique à 5 % pp de copolymère vinyl-2-pyridine co styrène, protégées contre la dégradation dans le rumen par une couche d'enrobage représentant 12 % en poids du produit protégé, composée d'un mélange de 20 % de copolymère vinyl-2-pyridine co styrène et de 80 % d'acide stéarique.

Le taux de protection des granulés est de 99 %.

### EXEMPLE COMPARATIF

2 % de granulés protégés préparés précédemment sont introduits dans un mélange de farines destiné à l'alimentation du bétail, et mis sous forme d'aliment granulé dans une presse à filière rotative KAHL aprés traitement à la vapeur, conformément à la méthode traditionnelle de préparation des aliments granulés. Le produit obtenu se présente sous forme de cylindres extrudés de 5 mm de diamètre et environ 15 mm de long.

Le taux de protection résiduel des acides aminés utilisés pour l'exemple, est de 0 % dans l'aliment granulé aprés passage dans la presse.

L'exemple comparatif montre que la mise sous forme d'aliment granulé par des méthodes traditionnelles de ce type de produit conduit à une dégradation totale de leur protection contre la dégradation dans le rumen, et par suite à la perte de leur efficacité pour l'alimentation du ruminant.

### EXEMPLES SELON L'INVENTION

### Exemple n°1:

Des pellets contenant des principes actifs qui sont détruits lors de la pelletisation et des granulés de principes actifs protégés contre la dégradation dans le rumen, et miscibles en toutes proportions à l'aliment granulé traditionnel, sont obtenus par mélange de granulés protégés décrits précédemment et de Bactérie lactique (Enterococcus faecium) avec un liant composé de:

| | |
|---|---|
| dolomie | 36,3 % |
| farine de soja | 9,0 % |
| principe actif protégé | 29,0 % |
| Enterococcus faecium contenant 3 10¹⁰ CFU/g | 3,1 % |
| eau | 12,9 % |
| lignosulfite à 42 % d'eau | 9,7 % |

Le mélange est réalisé à 20 °Celsius dans un mélangeur planétaire. Les constituants solides sont mélangés à sec puis le liant liquide est introduit. Dans ces conditions la pâte humide est réalisée très rapidement, le temps de mélange est ainsi sans influence sur la qualité du produit final.

Le mélange est mis sous forme de particules d'environ 8 mm de diamètre par extrusion, à 20 °Celsius, dans une presse laboratoire à piston de 70 mm de diamètre, entrainé par vis, à travers une filière comportant un cône de compression de 120 mm de longueur pour un diamètre initial de 70 mm et un orifice de 8 mm de diamètre.Les caractéristiques de ce matériel permettent d'éviter le bris des granulés de principe actif protégé.

Les pellets, ainsi obtenus, sont séchés en étuve ventilée, à 45 °Celsius en 5 heures, et réduits en éléments de longueur environ 20 mm.

Le taux de protection résiduel du principe actif protégé après maintien pendant 24 heures dans une solution à pH6 reste identique (99,4 %) dans le produit final. Le titre en acide aminé est de 19,2 %. Le taux de bactéries subsistantes est de 1,4 10⁸/g de produit sec. Le rendement opérationnel sur la numération des Bactéries non protégées selon la méthode de comptage ci-après est de 12,7 %.

On prend 100 g de granulés que l'on met dans 900 ml de tryptone sel, on agite. Cette solution constitue la solution de dilution -1, on effectue des dilutions successives de 1 ml dans 9 ml jusqu'à 10 ⁻⁹. Le milieu utilisé pour faire les dénombrements est le milieu MRS. On ensemmence dans la masse 1 ml des dilutions précédentes, on lit après incubation des boîtes 48 heures à 37°C.

### Exemple n°2:

On reproduit l'exemple 1 en mettant en oeuvre :

| | |
|---|---|
| dolomie | 36,1 % |
| farine de soja | 9,0 % |
| principe actif protégé | 27,3 % |
| Enterococcus faecium contenant 1,7 10¹⁰ CFU/g | 0,3 % |
| eau | 18,2 % |
| lignosulfite à 42 % d'eau | 9,1 % |

Le taux de protection 24 heures à pH6 résiduel du principe actif protégé reste identique (99,4 %) dans le produit final. Le titre en acide aminé est de 17,9 %.

Le taux de bactéries subsistantes est de 0,7 10⁸ CFU/g sur le produit sec. Le rendement sur cette opération sur la numération des Bactéries lactiques non protégées est de 20 %.

### Exemple 3:

On reproduit l'exemple 1 en introduisant :

| | |
|---|---|
| dolomie | 19,8 % |
| farine de soja | 9,9 % |
| principe actif protégé | 34,3 % |
| Enterococcus faecium contenant 3 10¹⁰ CFU/g | 9,3 % |
| eau | 14,5 % |
| lignosulfite à 42 % d'eau | 11,6 % |

Le taux de protection 24 heures à pH6 résiduel du principe actif protégé reste identique (99,4 %) dans le produit final. Le titre en acide aminé est de 18,8 %.

Le taux de bactéries subsistantes est de 3,9 10⁸ CFU/g. Le rendement opérationnel sur la numération des Bactéries lactique non protégées est de 11 %.

### Exemple 4:

On reproduit l'exemple 1 en introduisant:

| | |
|---|---|
| dolomie | 29,3 % |
| farine de soja | 7,5 % |
| principe actif protégé | 29,5 % |
| Enterococcus faecium contenant 3 10¹⁰ CFU/g | 11,8 % |
| eau | 11,5 % |
| lignosulfite à 42 % d'eau | 9,8 % |

Le taux de protection 24 heures à pH6 résiduel du principe actif protégé reste identique (99,4 %) dans le produit final. Le titre en acide aminé est de 19,4 %.

Le taux de bactéries subsistantes est de 5 10⁸ CFU/g. Le rendement opérationnel sur la numération des Bactéries lactiques non protégées est de 12 %.

### Exemple 5:

On reproduit l'exemple 1 en remplaçant la bactérie Enterococcus faecium par une souche de Pediococcus pentosaceus contenant 8 10¹⁰ CFU/g.

Le taux de protection 24 heures à pH6 résiduel du principe actif protégé reste identique (99,4 %) dans le produit final. Le titre en acide aminé est de 17,9 %.

Le taux de bactéries subsistantes est de 0,083 10⁸ CFU/g de ,produit sec. Le rendement sur cette opération sur la numération des Bactéries lactiques non protégées est de 0,28 %.

### Exemple 6:

On reproduit l'exemple 1 en remplaçant la bactérie Enterococcus faecium par une souche de Lactobacillus casei subsp.rhamnosus contenant 9,6 10⁹ CFU/g.

Le taux de protection 24 heures à pH6 résiduel du principe actif protégé reste identique (99,4 %) dans le produit final. Le titre en acide aminé est de 18,3 %.

Le taux de bactéries subsistantes est de 0,00029 10⁸ CFU/g. Le rendement opérationnel sur la numération des Bactéries lactique non protégées est de l'ordre de 0,01 %.

### Exemple 7:

On reproduit l'exemple 1 en modifiant les conditions de séchage qui sont de 5 heures à 55 ° Celsius.

Le taux de protection 24 heures à pH6 résiduel du principe actif protégé reste identique (99,4 %) dans le produit final. Le titre en acide aminé est de 19,8 %.

Le taux de bactéries subsisantes est de 0,4 10⁸ CFU/g. Le rendement opérationnel sur la numération des Bactéries lactique non protégées est de 3,6 %.

## Revendications

1. Compositions sous forme de "pellets" contenant des principes actifs non protégés vis à vis de la dégradation dans le rumen ; à l'exclusion d'un ou plusieurs des composés suivants : les dérivés hydrosolubles de la cellulose, les polysaccharides, les sucres, les mélasses, les vinasses, les lignosulfonates, les farines de grains ou d'algues, les composés minéraux cristallisables, les gélatines, les protéines tannées, les sels de cations polyvalent des polyacides, les huiles siccatives, les mastics, les acides ou alcools gras, les grasses animales et végétales hydrogénées, les esters de glycérol, les paraffines, les cires naturelles ou synthétiques, les polymères synthétiques, la silice, les silicates, le talc, les argiles, les carbonates de calcium, les phosphates, les résidus des industries des céréales et du bois, les tourteaux broyés, les résidus de brasserie et de fermentation, les fibres végétales cellulosiques ; en association avec des principes actifs granulaires de diamètre moyen compris entre 0,3 et 5 mm protégés vis-à-vis de la dégradation dans le rumen mais dégradables par l'action conjointe de la chaleur et de la pression lors de la pelletisation, utilisées pour la supplémentation alimentaire et/ou médicamenteuse des ruminants, libérant lesdits principes actifs protégés dans la caillette et/ou l'intestin caractérisées en ce qu'elles sont obtenues par mélange et mise en forme du ou des principes actifs non protégé(s) vis à vis du rumen, du principe actif protégé sous forme granulaire et d'un liant choisi parmi les liants solubilisables, réticulables ou fusibles et éventuellement d'un agent délitant et/ou d'une charge.

2. Compositions selon la revendication 1 caractérisées en ce que le principe actif protégé vis à vis du rumen est choisi parmi les vitamines, les aminoacides, les médicaments.

3. Compositions selon la revendication 2 caractérisées en ce que le principe actif protégé est choisi parmi les aminoacides, les vitamines, les médicaments protégés par un copolymère pH sensible, un produit naturel ou un produit dégradable par voie enzymatique.

4. Compositions selon la revendication 3 caractérisées en ce que le principe actif protégé est choisi parmi les aminoacides, les vitamines, les médicaments protégés par un copolymère pH sensible à base de vinyl-pyridine et de styrène en association avec une substance hydrophobe.

5. Compositions selon la revendication 3 caractérisées en ce que le principe actif protégé est choisi parmi les aminoacides, les vitamines, les médicaments protégés par un produit naturel ou un produit dégradable par voie enzymatique choisi parmi la zéïne ou le chitosan.

6. Compositions selon la revendication 1 caractérisées en ce que le principe actif non protégé vis à vis du rumen est choisi parmi les oligoéléments, les vitamines, les levures, les facteurs de croissance, les enzymes, la flore microbienne, les fungi, les peptides, le carbonate de sodium, le propylène glycol, la bétaïne, le propionate de sodium.

## Claims

1. Compositions in the form of "pellets" containing active ingredients unprotected against degradation in the rumen; with the exception of one or more of the following compounds: water-soluble cellulose derivatives, polysaccharides, sugars, molasses, vinasses, lignosulphonates, grain or algal meals, crystallizable inorganic compounds, gelatins, tanned proteins, polyvalent cation salts of polyacids, drying oils, mastics, fatty acids or alcohols, hydrogenated animal and vegetable fats, glycerol esters, paraffins, natural or synthetic waxes, synthetic polymers, silica, silicates, talc, clays, calcium carbonates, phosphates, residues of the cereal and timber industries, ground oil-cakes, brewery and fermentation residues, cellulosic plant fibres; combined with granular active ingredients with a mean diameter of between 0.3 and 5 mm, protected against degradation in the rumen but degradable through the combined action of heat and pressure during pelletisation, used for feed and/or medicinal supplementation for ruminants, which release the said protected active ingredients in the rennet-stomach and/or in the intestine, characterized in that they are obtained by mixing and shaping the active ingredient or ingredients unprotected with respect to the rumen, the active ingredient protected in granular form and a binder chosen from binders which can be solubilised, crosslinked or melted and, optionally, a disintegrating agent and/or a filler.

2. Compositions according to claim 1, characterised in that the active ingredient protected with respect to the rumen is chosen from vitamins, amino acids and medicinal products.

3. Composition according to claim 2, characterized in that the protected active ingredient is chosen from amino acids, vitamins and medicinal products protected by a pH-sensitive copolymer, a natural product or an enzyme-degradable product.

4. Compositions according to claim 3, characterized in that the protected active ingredient is chosen from amino acids, vitamins and medicinal products protected by a pH-sensitive copolymer based on vinylpyridine and styrene combined with a hydrophobic substance.

5. Compositions according to claim 3, characterized in that the protected active ingredient is chosen from amino acids, vitamins and medicinal products protected by a natural product or an enzyme-degradable product chosen from zein or chitosan.

6. Compositions according to claim 1, characterised in that the active ingredient unprotected with respect to the rumen is chosen from trace elements, vitamins, yeasts, growth factors, enzymes, microbial flora, fungi, peptides, sodium carbonate, propylene glycol, betaine and sodium propionate.

## Patentansprüche

1. Zusammensetzungen in Form von "Pellets", enthaltend gegenüber dem Abbau in dem Rumen ungeschützte Wirkstoffe unter Ausschluß von einer oder mehreren der folgenden Verbindungen: Wasserlösliche Cellulosederivate, Polysaccharide, Zucker, Melassen, Schlempen, Lignosulfonate, Korn- oder Algenmehle, kristallisierbare, mineralische Verbindungen, Gelatine, Gerbproteine, Salze mehrwertiger Kationen von Polysäuren, trocknende Öle, Mastix, Fettsäuren oder -alkohole, hydrierte tierische und pflanzliche Fette, Glycerinester, Paraffine, Natur- oder Synthesewachse, synthetische Polymere, Siliciumdioxid, Silicate, Talk, Tone, Calciumcarbonate, Phosphate, Rückstände der Getreide- und Holzindustrie, Preßrückstände, Brauerei- und Fermentationsrückstände, pflanzliche Cellulosefasern; in Assoziation mit granularen Wirkstoffen mit einem mittleren Durchmesser zwischen 0,3 und 5 mm, die gegenüber dem Abbau in dem Rumen geschützt, jedoch durch gemeinsame Einwirkung von Wärme und Druck während der Pelletisierung abbaubar sind, welche für die Nahrungsergänzung und/oder medikamentöse Ergänzung von Wiederkäuern verwendet werden, wobei die geschützten Wirkstoffe im Labmagen und/oder Darm freigesetzt werden, dadurch gekennzeichnet, daß sie durch Mischen und Formgebung des (der) gegenüber dem Rumen ungeschützten Wirkstoffs bzw. Wirkstoffe, des in granularer Form geschützten Wirkstoffs und eines Bindemittels, ausgewählt unter den solubilisierbaren, vernetzbaren oder schmelzbaren Bindemitteln, und gegebenenfalls eines Zerfallsmittels und/oder eines Füllstoffs erhalten werden.

2. Zusammensetzungen gemäß Anspruch 1, dadurch gekennzeichnet, daß der gegenüber dem Rumen geschützte Wirkstoff unter Vitaminen, Aminosäuren und Arzneimitteln ausgewählt ist.

3. Zusammensetzungen gemäß Anspruch 2, dadurch gekennzeichnet, daß der geschützte Wirkstoff unter den Aminosäuren, den Vitaminen, den durch ein pH-empfindliches Copolymeres geschützten Arzneimitteln, einem natürlichen Produkt oder einem auf enzymatischem Weg abbaubaren Produkt ausgewählt ist.

4. Zusammensetzungen gemäß Anspruch 3, dadurch gekennzeichnet, daß der geschützte Wirkstoff unter den Aminosäuren, den Vitaminen, den durch ein pH-empfindliches Copolymeres auf Basis von Vinylpyridin und Styrol geschützten Arzneimitteln in Assoziation mit einer hydrophoben Substanz ausgewählt ist.

5. Zusammensetzungen gemäß Anspruch 3, dadurch gekennzeichnet, daß der geschützte Wirkstoff ausgewählt ist unter den Aminosäuren, den Vitaminen, den Arzneimitteln, die geschützt sind durch ein natürliches Produkt oder ein auf enzymatischem Weg abbaubares Produkt, ausgewählt unter Zein oder Chitosan.

6. Zusammensetzungen gemäß Anspruch 1, dadurch gekennzeichnet, daß der gegenüber dem Rumen ungeschützte Wirkstoff unter den Oligoelementen, den Vitaminen, den Hefen, den Wachstumsfaktoren, den Enzymen, der mikrobiellen Flora, den Fungi, den Peptiden, Natriumcarbonat, Propylenglykol, Betain und Natriumpropionat ausgewählt ist.
